# EUROPEAN PATENT APPLICATION

(11) **EP 4 495 247 A1**
(43) Date of publication of application: **22.01.2025**
(21) Application number: 23770167.7
(22) Date of filing: 08.02.2023
(51) Int. Cl.: C12N 15/86, A61K 39/125, A61P 31/14, C07K 14/085, C07K 19/00, C12N 5/10, C12N 15/41

(54) **VIRUS-LIKE PARTICLE CONTAINING CAPSID PROTEINS CONNECTED BY LINKER**

(30) Priority: 18.03.2022 JP 2022043609
(71) Applicant: Denka Company Limited, Tokyo 103-8338 (JP)
(72) Inventor: INOMATA, Aoto, Tokyo 103-8338 (JP); MIKI, Motohiro, Tokyo 103-8338 (JP)
(74) Representative: Müller-Boré & Partner Patentanwälte PartG mbB
(86) International application number: PCT/JP2023/004126
(87) International publication number: WO 2023/176206

(57) **Abstract**

The present invention aims to provide a VLP that can have multivalent antigenicity and has two capsid proteins present in equal proportions. A virus-like particle, including an assembly of dimers of a first capsid protein and a second capsid protein, wherein the first capsid protein and the second capsid protein are connected to each other by a linker.

## Description

### TECHNICAL FIELD

The present application relates to capsid protein dimers connected by a linker, virus-like particles comprising the dimers, expression vectors and host cells for producing the virus-like particles, and a pharmaceutical composition comprising the dimers and/or the virus-like particles.

### BACKGROUND ART

When viruses infect other organisms (e.g., animals, plants, and bacteria), they cause various symptoms and diseases. Viral infection may be involved in, for example, colds, influenza, bronchitis, pneumonia, gastroenteritis, hepatitis, meningitis, rubella, cancer, and encephalitis in humans. In these days, when people are traveling more actively, the viral infection that was once limited to a specific region may spread to other regions. As such, even more effective measures against viral infection are demanded.

As one of the measures against the viral infection, vaccination is widely conducted. Vaccines stimulate immune function of a subject to enhance immunity against viruses, thereby preventing infection. Using excellent antigens is required to obtain highly effective vaccines. Accordingly, the development of antigens for vaccines has continued.

For example, PTL 1 discloses a chimeric viral protein 1 (VP1) composed of a shell (S)-domain of VP1 from a first Norovirus strain and a protruding (P)-domain that comprises at least a portion of a P-domain of VP1 from a second Norovirus strain, and virus-like particles (VLPs) composed of the VP1. Here, it is disclosed that a multivalent VLP is prepared by mixing different types of VLPs. In this technology, it is necessary to prepare VLPs for each antigen (target Norovirus strain), and then, as the number of target antigens increases, there are possibilities that preparing multivalent VLPs will require more time and effort, quality control will become more complicated, the amount of antigens will increase, the cost will increase, or the like.

On the other hand, PTL 2 discloses multivalent VLPs composed of antigenic proteins derived from two or more Norovirus genotypes. PTL 3 discloses that a multivalent VLP is prepared by mixing VP1s derived from different Norovirus genotypes.

### CITATION LIST

### PATENT LITERATURE

PTL 1: Japanese Translation of PCT International Application Publication No. 2013-533745
PTL 2: Japanese Translation of PCT International Application Publication No. 2010-505766
PTL 3: Japanese Patent Laid-Open No. 2010-539192

### SUMMARY OF INVENTION

### TECHNICAL PROBLEM

Regarding the preparation of VLPs having multivalent antigenicity, in conventional techniques, VLPs are formed by random self-assembly of a plurality of different VP1 monomers, and therefore, different VP1s may be present in the VLPs in different proportions. The present invention aims to provide VLPs that can have multivalent antigenicity and have two capsid proteins present in equal proportions.

### SOLUTION TO PROBLEM

In consideration of the above, the inventors of the present application conceived of connecting the first capsid protein and the second capsid protein by a linker, and hence, accomplished the present invention.

The present invention provides, but is not limited to, the following:
(1) A virus-like particle, comprising an assembly of dimers of a first capsid protein and a second capsid protein, wherein the first capsid protein and the second capsid protein are connected to each other by a linker.
(2) The virus-like particle according to (1), wherein the linker is a GS linker.
(3) The virus-like particle according to (2), wherein a C-terminus of the first capsid protein and an N-terminus of the second capsid protein are connected to each other by the GS linker.
(4) The virus-like particle according to (2) or (3), wherein the GS linker is a (GGGGS)ₙ linker, where n is an integer of 1 or more.
(5) The virus-like particle according to any of (1) to (4), wherein the first capsid protein and the second capsid protein are derived from the same virus or different viruses.
(6) The virus-like particle according to any of (1) to (5), wherein the first capsid protein and the second capsid protein are derived from viruses of the *Caliciviridae* family.
(7) A capsid protein dimer, wherein a first capsid protein and a second capsid protein are connected to each other by a linker.
(8) The capsid protein dimer according to (7), wherein the linker is a GS linker.
(9) The capsid protein dimer according to (8), wherein a C-terminus of the first capsid protein and an N-terminus of the second capsid protein are connected to each other by the GS linker.
(10) The capsid protein dimer according to (8) or (9), wherein the GS linker is a (GGGGS)ₙ linker, where n is an integer of 1 or more.
(11) The capsid protein dimer according to any of (7) to (10), wherein the first capsid protein and the second capsid protein are derived from the same virus or different viruses.
(12) The capsid protein dimer according to any of (7) to (11), wherein the first capsid protein and the second capsid protein are derived from viruses of the *Caliciviridae* family.
(13) A polynucleotide comprising a structure in which a polynucleotide that encodes a first capsid protein and a polynucleotide that encodes a second capsid protein are connected to each other through a polynucleotide that encodes a linker.
(14) The polynucleotide according to (13), wherein the linker is a GS linker.
(15) The polynucleotide according to (14), wherein the GS linker is a (GGGGS)ₙ linker, where n is an integer of 1 or more.
(16) The polynucleotide according to any of (13) to (15), wherein the first capsid protein and the second capsid protein are derived from the same virus or different viruses.
(17) The polynucleotide according to any of (13) to (16), wherein the first capsid protein and the second capsid protein are derived from viruses of the *Caliciviridae* family.
(18) An expression vector comprising the polynucleotide according to any of (13) to (17).
(19) A host cell, into which the expression vector according to (18) is introduced.
(20) A pharmaceutical composition comprising the virus-like particle according to any of (1) to (6), or the capsid protein dimer according to any of (7) to (12).
(21) The pharmaceutical composition according to (20), wherein the pharmaceutical composition is used for induction of protective immunity.
(22) The pharmaceutical composition according to (20) or (21), wherein the pharmaceutical composition is a vaccine.

### BRIEF DESCRIPTION OF DRAWINGS

[Fig. 1] Fig. 1 shows an example of a conceptual diagram of a dimer of capsid proteins connected to each other by a linker.
[Fig. 2] Fig. 2 shows results by SDS-PAGE. Marker: a molecular weight marker, Washington0207: a control (VP1 of Washington0207 without a linker) sample, Aomori2-Washington0207: a sample containing a dimer of Aomori2 VP1 and Washington0207 VP1 connected by a linker, (G₄S)₄₋₆: a linker with 4 to 6 repeating units. Upper arrow: a band around 120 kDa, lower arrow: a band around 60 kDa.
[Fig. 3] Fig. 3 shows results by Western blotting. Marker: a molecular weight marker, Washington0207: a control (Washington0207 VP1 without a linker) sample, Aomori2-Washington0207: a sample containing a dimer of Aomori2 VP1 and Washington0207 VP1 connected by a linker, (G₄S)₄₋₆: a linker with 4 to 6 repeating units. Upper arrow: a band around 120 kDa, lower arrow: a band around 60 kDa.
[Fig. 4] Fig. 4 shows analysis results by a transmission electron microscope (TEM).
[Fig. 5] Fig. 5 shows results by SDS-PAGE. Marker: a molecular weight marker, Aomori2: a control (Aomori2 VP1 without a linker) sample, Aomori2-Washington0207 (G₄S)₅: a sample containing a dimer of Aomori2 VP1 and Washington0207 VP1 connected by a linker, (G₄S)₅: a linker with 5 repeating units.
[Fig. 6] Fig. 6 shows results by Western blotting. Marker: a molecular weight marker, Aomori2: a control (Aomori2 VP1 without a linker) sample,
   Aomori2-Washington0207 (G₄S)₅: a sample containing a dimer of Aomori2 VP1 and Washington0207 VP1 connected by a linker, (G₄S)₅: a linker with 5 repeating units.
[Fig. 7] Fig. 7 shows analysis results of a linker-connected VLP by a transmission electron microscope (TEM).
[Fig. 8] Fig. 8 shows an analysis result of the linker-connected VLP by size-exclusion chromatography (SEC).
[Fig. 9] Fig. 9 shows an analysis result of a comparable control sample (Aomori2 VLP) by size-exclusion chromatography (SEC).
[Fig. 10] Fig. 10 shows results of receptor binding-inhibitory activity against each VLP, when using mouse immune serum against GII.4 Aomori2 VLP. BT50 in the figure is an average value of BT50s obtained by using 4 lots of mouse immune serum. The results obtained by using each lot are shown as an open circle (O). Aomori2: Aomori2 VLP, Washington0207: Washington0207 VLP, Aomori2-Washington0207: a linker-connected VLP of Aomori2-Washington0207. *: p<0.05 (Whitney U test).
[Fig. 11] Fig. 11 shows results of receptor binding-inhibitory activity against each VLP, when using mouse immune serum against GII.4 Washington0207 VLP. BT50 in the figure is an average value of BT50s obtained by using 4 lots of mouse immune serum. The results obtained by using each lot are shown as an open circle (O). Aomori2: Aomori2 VLP, Washington0207: Washington0207 VLP, Aomori2-Washington0207: a linker-connected VLP of Aomori2-Washington0207. *: p<0.05 (Whitney U test).

### DESCRIPTION OF EMBODIMENTS

### <Virus-Like Particle, and Capsid Protein Dimer>

The present invention provides a virus-like particle (hereinafter, also referred to as "VLP"). Virus-like particles have a structure similar to that of virus particles. On the other hand, virus-like particles are different from virus particles in that they do not have genetic information (DNA or RNA) inside, and thereby they do not have the ability to infect or replicate. For this reason, virus-like particles can be used as antigens for vaccines against viral infection.

The virus-like particle of the present invention has a first capsid protein and a second capsid protein. Assembling the first capsid protein and the second capsid protein allows the virus-like particle to be composed. In the present invention, the first capsid protein and the second capsid protein form a dimer, and assembling a plurality of such dimers allows the virus-like particle to be composed. In other words, the virus-like particle of the present invention can be said to be an assembly of dimers of the first capsid protein and the second capsid protein. Accordingly, the present invention also provides such dimers. The number of dimers constituting the virus-like particles is not limited. It can be understood that an arbitrary number of dimers spontaneously assemble to form a structurally more stable virus-like particle. For example, a virus-like particle can be an assembly of 90 dimers.

The dimer of the present invention has a structure in which the first capsid protein and the second capsid protein are connected to each other by a linker. Here, the linker may be any linker as long as the object of the present invention can be achieved. Also, the structure and repeating unit of the linker, the mode of connection with the capsid proteins, and the like can be appropriately set depending on the capsid proteins that constitute the dimer. For example, the repeating unit of the linker may be set to one or more, preferably, 2, 3, 4, 5, 6, 7, 8, 9, or 10. For example, the linker can connect the first capsid protein and the second capsid protein at an arbitrary position such as their amino (N) termini, carboxyl (C) termini, side-chain amino groups and/or side-chain carboxyl groups. The arbitrary position can preferably be any position except for positions that may be included in an epitope region or that may affect the conformation of the epitope region.

Examples of the linker include peptide linkers. A peptide linker can include an arbitrary number of arbitrary amino acids. For example, the peptide linker can be composed of 3, 4, 5, 6, 7, 8, 9, 10, 11, 12, 13, 14, or 15 amino acids. Preferably, the peptide linker is composed of 3, 4, 5, 6, or 7 amino acids. A GS linker comprising glycine (G) and serine (S) is an example of a preferred peptide linker in the present invention. In addition, a linker that has a flexibility similar to the GS linker can be used.

A method for connecting the first capsid protein and the second capsid protein by the linker is not particularly limited. For example, the first capsid protein and the second capsid protein may be connected to each other by the linker at the arbitrary position in a chemical manner. Alternatively, a genetic engineering approach can be used. A polynucleotide that encodes the first capsid protein and a polynucleotide that encodes the second capsid protein are connected to each other by a polynucleotide that encodes a linker to obtain a dimer in which the first capsid protein and the second capsid protein are connected to each other by the linker, from the connected polynucleotide by using an appropriate expression system. Methods for connecting the linker to the peptides of proteins are known to those skilled in the art. Therefore, in the present invention as well, the first capsid protein and the second capsid protein can be connected to each other by the linker using any known method, without limitation by the above example.

For example, by using (G₄S)ₙ or (GGGGS)ₙ (SEQ ID NO: 1) as the GS linker, the first capsid protein and the second capsid protein can be connected to each other. Here, n is a repeating unit of the linker and an integer of 1 or more. Preferably, n is an integer from 2 to 8, more preferably from 4 to 6, and further preferably 5. The GS linker can connect the first capsid protein and the second capsid protein at an arbitrary position as long as a virus-like particle can be formed. The arbitrary position can preferably be any position except for positions that may be included in an epitope region or that may affect the conformation of the epitope region. For example, the C-terminus of the first capsid protein and the N-terminus of the second capsid protein can be connected to each other by the linker. For example, the N-terminus of the first capsid protein and the C-terminus of the second capsid protein can be connected to each other by the linker.

The first capsid protein and the second capsid protein can be derived from any viruses. Here, the viruses are not particularly limited, and may be, for example, viruses from the same or different families, genera, or species. In general, since capsid proteins from viruses of the same genus may have similar conformation, any capsid proteins can be used as long as they are derived from viruses of the same genus. Alternatively, the viruses may be of the same or different genogroups, genotypes, or serotypes. Also, the viruses may infect any host. Examples thereof include bacteriophages that infect bacteria, plant viruses that infect plants, and animal viruses that infect animals. More specifically, it is a virus of the *Caliciviridae* family. The *Caliciviridae* family include the genus Lagovirus, the genus Norovirus, the genus Hepevirus, the genus Sapovirus, and the genus Nebovirus. Note that information on the amino acid sequences of capsid proteins of the viruses can be obtained from publicly available information sources. Examples thereof include databases published on the Internet or the like (e.g., without limitation, GenBank, EMBL, and DNA Data Bank of Japan (DDBJ)).

When the first capsid protein and the second capsid protein are both selected from capsid proteins derived from viruses of the same family, genus, or species, the resultant dimer is composed of two capsid proteins which are the same or different. Therefore, the virus-like particles formed by assembling such dimers have mono- or di-valent antigens of viruses of the same family, genus, or species. When the first capsid protein and the second capsid protein are each selected from capsid proteins derived from viruses of different families, genera, or species, the resultant dimer is composed of two different capsid proteins. Therefore, the virus-like particles formed by assembling such dimers have divalent antigens of viruses of the different families, genera, or species.

Also, when the first capsid protein and the second capsid protein are both selected from capsid proteins derived from viruses of the same genogroup, genotype, or serotype, the resultant dimer is composed of two capsid proteins which are the same or different. Therefore, the virus-like particles formed by assembling such dimers have mono- or di-valent antigens of viruses of the same genogroup, genotype, or serotype. When the first capsid protein and the second capsid protein are each selected from capsid proteins derived from viruses of different genogroups, genotypes, or serotypes, the resultant dimer is composed of two different capsid proteins. Therefore, the virus-like particles formed by assembling such dimers have divalent antigens of viruses of the different genogroups, genotypes, or serotypes.

From the above description, it can be understood that the dimers of the present invention comprise the first and second capsid proteins in equal proportions (1:1). Also, it can be understood that the virus-like particles of the present invention, which are formed by assembling the dimers, comprise the first and second capsid proteins in equal proportions (1:1), and further, they may have a regular or uniform structure with the dimers as a unit. This fact can be difficult to achieve with conventional techniques, in which monomers of capsid proteins are mixed to form virus-like particles by their spontaneous assembly. VLPs of conventional techniques are a mixture of VLPs having different proportions of capsid proteins and may not have a uniform structure.

The first and second capsid proteins composing the virus particles and the dimers of the present invention will be described in more detail, using a case where these proteins are derived from Norovirus as an example. It should be understood that the present invention can also apply to capsid proteins derived from other viruses.

Noroviruses are known to have 10 genogroups (GI, GII, GIII, GIV, GV, GVI, GVII, GVIII, GIX, GX). It is known that Noroviruses belonging to GI, GII, GIV, GVIII, and GIX infect humans, Noroviruses belonging to GIII infect cattle, Noroviruses belonging to GV infect mice, Noroviruses belonging to GVI infect cats, Noroviruses belonging to GVII infect dogs, and Noroviruses belonging to GX infect bats. Each genogroup has genotypes that are classified or clustered based on gene sequences of Noroviruses. For example, as for GI and GII, it is known that there are various genotypes. At present, GI includes 9 genotypes (GI.1 to 9), and GII includes 27 genotypes (GII.1 to 27). Noroviruses include, but are not limited to, Norwalk (M87611), Southampton (L07418), DesertShield395 (U04469), Chiba407 (AB042808), Musgrove (AJ277609), BS5 (Hesse) (AF093797), Winchester (AJ277609), Boxer (AF538679), Vancouver730 (HQ637267), Hawaii (U07611), Melksham (X81879), SnowMountain (AY134748), Ibaraki197 (LC213885), MK04 (DQ456824), Hubei027 (MH068811), HuzhouNS17116 (MG763368), 218001 (MK614154), TV24 (U02030), NS17-A863 (MG892947), NS17-A928 (MG892950), NS17-A1335 (MG892956), Bristol (X76716), Hillingdon (AJ277607), Seacroft (AJ277620), DingHai30 (MH068811), GZ2010-L96 (JX989075), NORO_173 (MH218642), PA226 (MH114014), 15-BA11 (MH279838), 016Q01 (KY407213), Leeds (AJ277608), Amsterdam (AF195848), VA97207 (AY038599), Erfurt546 (AF427118), Sw918 (AB074893), Wortley (AJ277618), M7 (AY130761), Tiffin (AY502010), CS-E1 (AY502009), Kawasaki308 (LC037415), OH-QW101 (AY823304), OH-QW170 (AY823306), Luckenwalde591 (EU373815), IF1998 (AY675554), Yuri (AB083780), Loreto1847 (KT290889), Loreto1972 (KY225989), Beijin53931 (GQ856469), Leon4509 (KU306738), Loreto0959 (MG495077), Loreto1257 (MG495079), PNV06929 (MG706448), CHDC5191 (ACT76139), Camberwell (AF145896), Lordsdale (X86557), Grimsby (AJ004864), Miami Beach (AF414424), Farmington Hills (AY502023), Houston (EU310927), Chiba04-1050 (AB220921), Hunter504D (DQ078814), DenHaag89 (EF126965), Saga1 (AB447456), Aomori2 (AB447433), Yerseke38 (EF126963), Apeldoorn317 (AB445395), Osaka1 (AB541319), OC07138 (AB434770), NewOrlens1805 (GU445325), Sydney/NSW0514 (JX459908), Washington0207 (MK754446), and CUHK-NS-2200 (MN400355) (genome accession Nos. in brackets).

In the present invention, the first and second capsid proteins can each be selected from ones derived from any Norovirus, and can be arbitrarily combined. The first and second capsid proteins can be selected from, for example, but are not limited to, capsid proteins derived from Noroviruses GI and GII. More specifically, the first and second capsid proteins can each be selected from capsid proteins derived from Norovirus GI or GII. Alternatively, one of the first and second capsid proteins can be selected from capsid proteins derived from Norovirus GI, and the other can be selected from capsid proteins derived from Norovirus GII. Here, as Norovirus GI, any of GI.1 to 9 can be used, and as Norovirus GII, any of GII.1 to 27 can be used. As a further example, a capsid protein derived from Norovirus GII.4 can be selected as the first capsid protein, and a capsid protein derived from Norovirus GII.17 can be selected as the second capsid protein. Also, capsid proteins derived from Norovirus GII.4 or GII.17 can be selected as the first and second capsid proteins. Needless to say, the first capsid protein and the second capsid protein are interchangeable with each other. Since capsid proteins derived from Norovirus may have similar conformation between genogroups, genotypes, or strains, not only the above-mentioned combination of the first and second capsid proteins, but any combination can be adopted. Here, the capsid protein can comprise VP1 capsid protein. Alternatively, the capsid protein can consist of VP1 capsid protein. Polypeptides (SEQ ID NOs: 10 to 12) in which VP1 capsid proteins of Aomori2 and Washington0207 of Norovirus are connected to each other by a linker (4 to 6 repeating units) are specific examples of components of the capsid protein dimer of the present invention and the virus-like particle of the present invention.

### <Polynucleotide, Expression Vector, and Host Cell>

The present invention provides a polynucleotide that encodes the above-mentioned capsid protein dimers. Correspondence between amino acid residues and gene codons has already been established, and therefore, the amino acid sequences of the dimers and corresponding polynucleotide sequences are easily mutually converted. The polynucleotide can be either RNA or DNA, but DNA is convenient from the viewpoint of the aspects of handling and storage.

The polynucleotide that encodes the capsid protein dimer of the present invention can be obtained by biological techniques. For example, RNA that encodes capsid proteins of viruses is reverse-transcribed into cDNA, gene amplification reaction (reverse transcription-PCR) is performed using the cDNA as a template, and thereby polynucleotides that encode the first and second capsid proteins can respectively be amplified. Primers for PCR are designed such that a region including a nucleic acid sequence that encodes the first or second capsid protein are amplified. In other words, primers can be designed that anneals to a nucleic acid sequence located upstream and/or downstream of the nucleic acid sequence that encodes the first or second capsid protein.

Further, the sequence information on the polynucleotides that encode capsid proteins of viruses can also be obtained from publicly available information sources. Examples thereof include databases published on the Internet or the like (e.g., without limitation, GenBank, EMBL, and DNA Data Bank of Japan (DDBJ)).

Alternatively, the polynucleotide that encodes the capsid protein dimer of the present invention can be obtained by chemical synthesis. Polynucleotide synthesis can be performed in-house, or can be outsourced to an external organization. Polynucleotides can be synthesized by any known method. For example, the solid-phase synthesis methods are well-known. Those skilled in the art are thoroughly familiar with the methods and conditions for polynucleotide synthesis.

From the polynucleotide that encodes the first capsid protein and the polynucleotide that encodes the second capsid protein that have been obtained by a method as described above, the first capsid protein and the second capsid protein can be produced by using an appropriate expression system.

Here, in a case where the first capsid protein and the second capsid protein are produced in a separate expression system, the dimer of the present invention can be obtained by chemically connecting the resultant first capsid protein and second capsid protein by a linker, as described above.

Alternatively, when a peptide linker is used as the linker, a polynucleotide can be prepared by connecting a polynucleotide that encodes the first capsid protein and a polynucleotide that encodes the second capsid protein to each other through a polynucleotide that encodes the peptide linker. Peptide linkers such as those described above can be used. An Example of the peptide linker is a GS linker (G₄S)ₙ or (GGGGS)ₙ linker (where, n is as defined above). By using such as polynucleotide, a dimer in which the first capsid protein and the second capsid protein are connected to each other by a linker can be obtained as a product. According to such a technique, the first capsid protein and the second capsid protein are not required to be chemically connected to each other by a linker, after separately producing them.

The polynucleotide described above can be subjected to operations of extraction and/or purification as necessary, at any time during its acquisition or utilization. Those skilled in the art can appropriately select a known method for extraction and/or purification of the polynucleotide.

Polynucleotides of SEQ ID NOs: 7 to 9 encode a polypeptide in which VP1 capsid proteins of Aomori2 and Washington0207 of Norovirus are connected to each other by a linker (4 to 6 repeating units), and are specific examples of the polynucleotide that encodes the capsid protein dimer of the present invention.

The polynucleotide that encodes the capsid protein dimer of the present invention can be incorporated into an expression vector. Accordingly, the present invention also provides such an expression vector. Examples of the expression vector include pET for E. coli expression, pAUR for yeast expression, pIEx-1 for insect cell expression, and pBApo-CMV for animal cell expression, but other known vectors can also be used. Note that the incorporation of the polynucleotide into the expression vector can be performed by a known method.

The expression vector of the present invention can be introduced into a suitable host cell. Accordingly, the present invention provides a host cell into which the expression vector is introduced. The host cell is not particularly limited as long as it can produce the capsid protein dimer of the present invention. For example, insect-derived cells (e.g., Sf9, and High Five cells), E. coli, yeast (e.g., *Saccharomyces cerevisiae, Saccharomyces pombe,* and *Pichia pastoris),* mammalian cells (e.g., CHO, and HEK), and other any cells can be used as the host cell. By culturing the host cell of the present invention, the capsid protein dimer of the present invention is produced, and the dimer is assembled to form the virus-like particle of the present invention. Note that methods for introducing the expression vector into the host cell, culturing the host cell, and extracting, concentrating or purifying the product are known. Such known methods can also be applied to the present invention.

### <Pharmaceutical Composition>

The present invention provides a pharmaceutical composition comprising the virus-like particle or the capsid protein dimer of the present invention described above.

The pharmaceutical composition of the present invention may comprise any additional constituents as long as the effects of the present invention are achieved. Additional constituents can be appropriately selected from known ones. Examples thereof include excipients, diluents, pH adjusters, preservatives, carriers, suspending agents, solubilizers, thickeners, stabilizers, antiseptic agents, penetrants, immune modulators, and adjuvants.

The pharmaceutical composition of the present invention may be for oral administration or parenteral administration, and the intended administration route can be appropriately selected. The pharmaceutical composition of the present invention can be administered by any route, including venous, arterial, muscular, peritoneal, nasal, transdermal, intradermic, subcutaneous, buccal, sublingual, rectal, mouth, ocular, vaginal, pulmonary, and oral routes.

The form of the pharmaceutical composition of the present invention is not limited, and can be, for example, tablets, capsules, pills, syrup, elixirs, emulsions, aerosol, aqueous or non-aqueous injection solutions, or powders, granules, or tablets for injection solutions (can be made by adding liquid excipients, aqueous or non-aqueous, to prepare injection solutions).

The pharmaceutical composition of the present invention may treat (prevent, cure, alleviate, or improve) a viral infection, a virus-induced disease, or at least one symptom related to the infection and/or the disease. The above description applies to the viruses used herein. In addition, examples of the infections, diseases, or symptoms include acute gastroenteritis and its related symptoms (at least one of nausea, diarrhea, loose stool, vomiting, sicchasia, fever, malaise, fatigue, gastrospasm, ague, muscular pain, headache). Still, it should be understood that they are not limited to those listed above. The prevention, treatment, alleviation, or improvement described above can be achieved by neutralizing infection agents, inhibiting the entry of infectious agents into cells, inhibiting the replication of infectious agents, preventing host cells from being infected or destroyed, or stimulating antibody production, but is not limited to these manners.

The pharmaceutical composition of the present invention can be administered to a subject at an effective amount, in order to prevent, treat, alleviate, or improve a viral infection, a virus-induced disease, or at least one symptom related to the infection and/or the disease. Any subject may be a target as long as they can be infected with a virus, and examples thereof can include mammals (humans, pigs, cattle, rodents, dogs, cats, and the like). The effective amount can be appropriately set, taking into consideration of the subjects to whom the composition is applied, the administration routes, the administration forms, or the like.

In addition, the pharmaceutical composition of the present invention induces the protective immunity against viruses, and thereby, it can be used in order to prevent, treat, alleviate, or improve a viral infection, a virus-induced disease, or at least one symptom related to the infection and/or the disease. The induction of "protective immunity" against viruses used herein means inducing immunity or immune responses against infection agents (viruses, substances derived therefrom, or substances produced thereby). The protective immune response may result from either an humoral immune response or a cell-mediated immune response. When the protective immunity against a virus is induced, the antibody titer against the virus may increase in the subject from before the induction. The measurement of antibody titers can be performed by a known method. Accordingly, the pharmaceutical composition of the present invention can be used as a composition for vaccines.

The pharmaceutical composition of the present invention can be used to treat, for example, infections, diseases, and symptoms related to Norovirus. Norovirus is as described above. Norovirus targeted by the pharmaceutical composition of the present invention is not limited, and it may belong to any genogroup or genotype. However, it should be understood that Norovirus to be targeted is determined by the source of the virus-like particles, or the capsid proteins that constitute the dimer comprised in the pharmaceutical composition of the present invention. For example and without limitation, when the virus-like particles or the capsid protein dimer comprised in the pharmaceutical composition comprise:
- capsid proteins derived from Norovirus GI, the target of the pharmaceutical composition is at least Norovirus GI;
- capsid proteins derived from Norovirus GII, the target of the pharmaceutical composition is at least Norovirus GII; and
- proteins derived from Norovirus GI and GII, the targets of the pharmaceutical composition are at least Norovirus GI and GII.

The virus-like particle of the present invention and the capsid protein dimer of the present invention may have a regular or uniform structure comprising the first and second capsid proteins in equal proportions (1:1) as described above. This feature is useful for the quality control of the pharmaceutical composition. Also, when the virus-like particle of the present invention or the capsid protein dimer of the present invention has di- or multi-valent antigenicity, this can be useful for efficiently producing a pharmaceutical composition that has multivalent antigenicity. This can also be useful in enabling the amount added as proteins to be reduced.

### EXAMPLES

The present invention will be described in more detail by the following Examples. The examples are provided for the purpose of better understanding the present invention, and are not intended to limit the scope of the present invention.

### [Example 1] Preparation of Recombinant Baculovirus

The following VP1 genes derived from Norovirus strains were used:
(1) VP1 gene derived from Norovirus Hu/GII-4/Aomori2/2006/JP (Accession number: AB447433) (hereinafter, referred to as "Aomori2") (SEQ ID NO: 2).
(2) VP1 gene derived from Norovirus GII isolate Hu/US/2018/GII.P16-GII.4 Sydney/Washington0207 (Accession number: MK754446) (hereinafter, referred to as "Washington0207") (SEQ ID NO: 3).

The G₄S linkers were used as the linker, and their repeating units were each set to 4 ((G₄S)₄), 5 ((G₄S)₅), and 6 ((G₄S)₆). A gene that encodes (G₄S)₄ (SEQ ID NO: 4), a gene that encodes (G₄S)₅ (SEQ ID NO: 5), and a gene that encodes (G₄S)₆ (SEQ ID NO: 6) were obtained.

DNA in which two different VP1 genes were connected by the linker was obtained by gene synthesis. DNA in which two VP1 genes were connected by the gene that encodes (G₄S)₄ linker (SEQ ID NO: 7), DNA in which two VP1 genes were connected by the gene that encodes (G₄S)₅ linker (SEQ ID NO: 8), and DNA in which two VP1 genes were connected by the gene that encodes (G₄S)₆ linker (SEQ ID NO: 9) were obtained.

These DNAs were each incorporated into a transfer vector (pFastBac (trademark) 1 vector, Thermo Fisher Scientific, Inc., Cat. No. 10360014). Next, the transfer vector was introduced into E. coli that carries baculovirus genome DNA (MAX Efficiency (trademark) DH10Bac Competent Cells, Thermo Fisher Scientific, Inc., Cat. No. 10361012), and DNA of interest was incorporated into baculovirus genome DNA by homologous recombination. Baculovirus genome DNA was extracted and purified from E. coli (QIAprep Spin Miniprep Kit, QIAGEN N.V., Cat. No. 27106), and introduced into Sf9 cells (Lipofectamine (trademark) LTX Reagent with PLUS (trademark) Reagent, Thermo Fisher Scientific, Inc., Cat. No. 15338100). After the Sf9 cells were cultured at 26 to 28°C for 7 days, recombinant baculovirus was collected from the culture supernatant and stored at -80°C.

### [Example 2] Preparation of Linker-Connected VLP

The recombinant baculovirus prepared in Example 1 was inoculated into High Five cells at an appropriate multiplicity of infection (MOI). The High Five cells were cultured at 26 to 28°C to produce linker-connected VLPs. The culture solution was collected 4 to 7 days after infection, centrifuged at 10,000 ×g for 60 minutes, and separated into the culture supernatant and cell pellet. The cell pellets were crushed with an ultrasonic crusher, and centrifuged at 15,000 ×g for 10 minutes to collect the supernatant. The culture supernatant and the supernatant after crushing the cell pellets were subjected to density gradient centrifugation using cesium chloride to obtain linker-connected VLPs.

### [Example 3] Characterization of VLP

The expression of the dimer of linker-connected VP1 proteins, which are components of linker-connected VLPs, was confirmed using SDS-PAGE and Western blotting.

SDS-PAGE analysis was performed as follows. 27 µl of the sample prepared in Example 2 and 9 µl of a sample buffer (4 × Laemmli Sample Buffer, Bio-Rad Laboratories, Inc., Cat. No. #161-0747) (containing DTT) were mixed and heated at 95°C for 5 minutes. 12 µl of the heated solution was applied to one lane of SDS-PAGE gel and subjected to electrophoresis at 200 V for 30 minutes. The gel after electrophoresis was stained with Coomassie (Bio-Safe Coomassie Stain, Bio-Rad Laboratories, Inc., Cat. No. #1610787) followed by the decolorization with D. W. for 20 minutes × 3 times.

Western blotting analysis was performed as follows. Using a transfer apparatus, the proteins on the gel after the above SDS-PAGE were transferred to a PVDF membrane (200 V, 30 minutes). After immersion treatment with a blocking buffer, an antibody for GII.4 genotype detection (anti-VLP antibody of Norovirus Hu/GII.4/Sydney/NSW0514/2012/AU (JX459908)) was diluted 10,000-fold and used to detect GII.4 genotype VP1.

Each gel image was obtained by using a gel photography device. And then, the densities of the resultant bands were quantified by the analysis panel to calculate the purity of the VLPs.

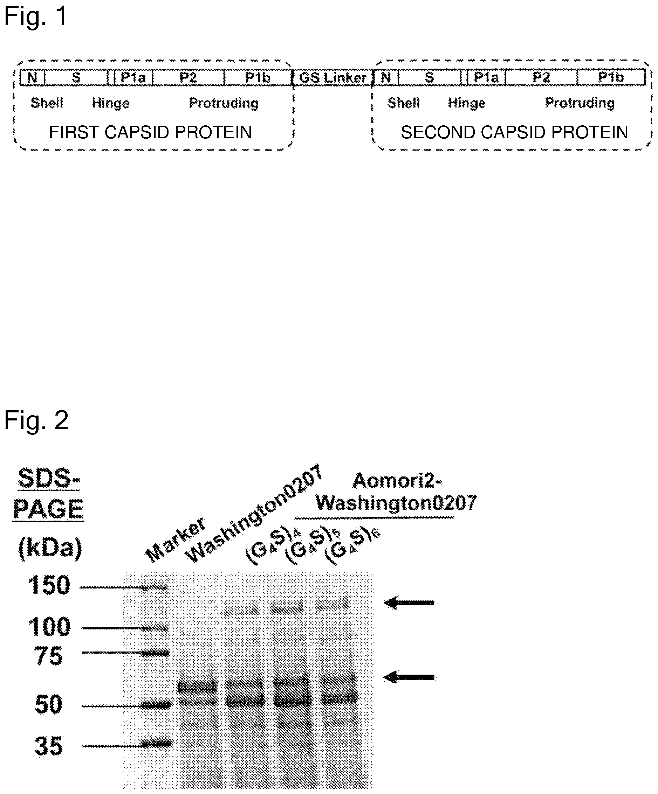

The results of SDS-PAGE analysis are shown in Fig. 2. In the sample obtained by using the recombinant baculovirus prepared in Example 1, a band was detected around 120 kDa (the lane of "Aomori2-Washington0207" in Fig. 2). In the comparative control sample (the lane of "Washington0207" in Fig. 2), no band was detected around 120 kDa. Bands that seem to correspond to VP1 monomer proteins were detected around 60 kDa, and hence, suggesting that the 120 kDa band was a protein in which 2 VP1s (VP1 derived from Aomori2 and VP1 derived from Washington0207) were connected by a linker. Also, bands around 120 kDa were observed regardless of the length of the linker used.

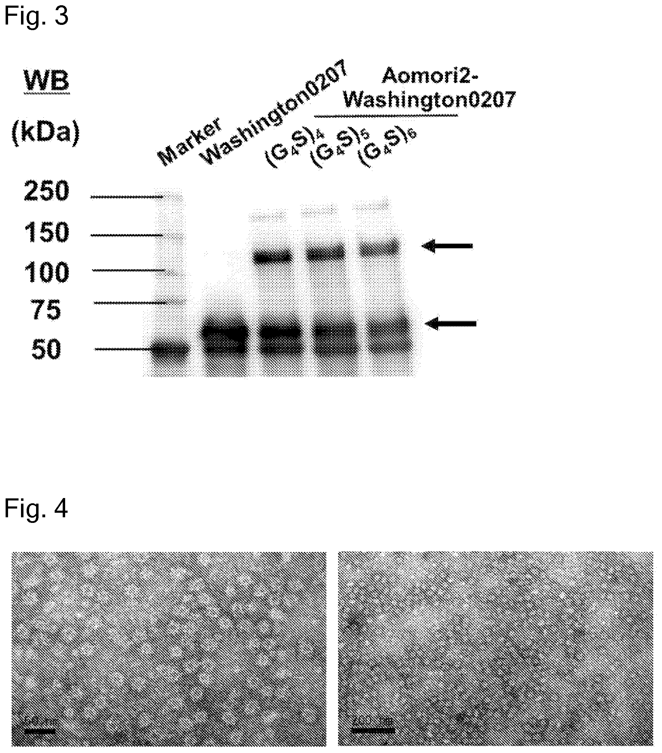

The results of Western blotting analysis are shown in Fig. 3. The bands detected around 60 kDa were confirmed to correspond to the VP1 monomer. The bands were detected at the same position in both cases where VP1 is derived from Washington 0207 and derived from Aomori2. The bands detected around 120 kDa were confirmed to be a protein in which 2 VP1s were connected by a linker (the lane of "Aomori2-Washington0207" in Fig. 3). In the comparative control sample (the lane of "Washington0207" in Fig. 3), no band was detected around 120 kDa. Also, the expression of a protein in which 2 VP1s were connected by a linker was observed regardless of the length of the linker used, and hence, it was found that the expression of the connected protein does not depend on the length of the linker.

### [Example 4] Analysis with Transmission Electron Microscope (TEM)

The structural characteristics of the linker-connected VP1 dimer protein in the sample prepared in Example 2 were analyzed using TEM.

TEM analysis was performed as follows. A drop of the sample after dilution was placed on a copper mesh and colored with a staining solution of phosphotungstic acid for observation with an electron microscope.

As a representative example, the analysis results of the (G₄S)₅ linker-connected VP1 dimer protein are shown in Fig. 4. From the drawing, the linker-connected VP1 dimer was confirmed to form particles (VLP). This result suggested that the VLP was a chimeric VLP having multivalent antigenicity against 2 Norovirus strains (Aomori2 and Washington0207). Similarly, in the case of using other linkers, it is presumed that linker-connected VP1 dimer proteins form VLPs.

### [Example 5] Analysis with Size-Exclusion Chromatography (SEC)

The structural characteristics of the linker connected VP1 dimer protein in the sample prepared in Example 2 were analyzed using SEC.

The SEC conditions are as follows. 50 µl sample of the purified linker-connected VP1 dimer protein was injected. The flow rate was set to 0.8 ml/min, the column used was TSKgel G6000PWX L(Tosoh Bioscience, Inc., Cat. No. 0008024). As a sample dilution and mobile phase, a phosphate buffer solution (pH=7.4) was used.

By SEC analysis as well, the linker-connected VP1 dimer protein was confirmed to form particles.

### [Example 6] Repreparation and Characterization of Linker-Connected VLP

The recombinant baculovirus prepared in Example 1 was inoculated at an appropriate multiplicity of infection (MOI) into High Five cells cultured at 26 to 28°C. The culture solution was collected 4 to 7 days after inoculation, and centrifuged at 10,000 ×g for 20 minutes to collect the cell pellets. The cell pellets were crushed with an ultrasonic crusher, centrifuged at 15,000 ×g for 10 minutes to collect the supernatant. The collected supernatant was subjected to sucrose cushion centrifugation (40% w/w sucrose solution) to collect pellets containing the linker-connected VLPs from which low molecular weight impurities were removed. The cell pellets were suspended in PBS and subjected to cesium chloride density gradient centrifugation to obtain purified linker-connected VLPs.

As the characterization of the obtained linker-connected VLPs, SDS-PAGE and Western blotting in the same manner as in Example 3, electron microscopy observation (TEM) in the same manner as in Example 4, and size-exclusion chromatography (SEC) were performed to confirm particle formation. Further, multi-angle light scattering (MALS) was performed to evaluate particle sizes.

SEC was performed according to the conditions in Example 5 except that 20 µl of the sample was injected. Peak fractions that were separated and detected by SEC were subjected to MALS to measure the particle sizes at dn/dc = 0.185.

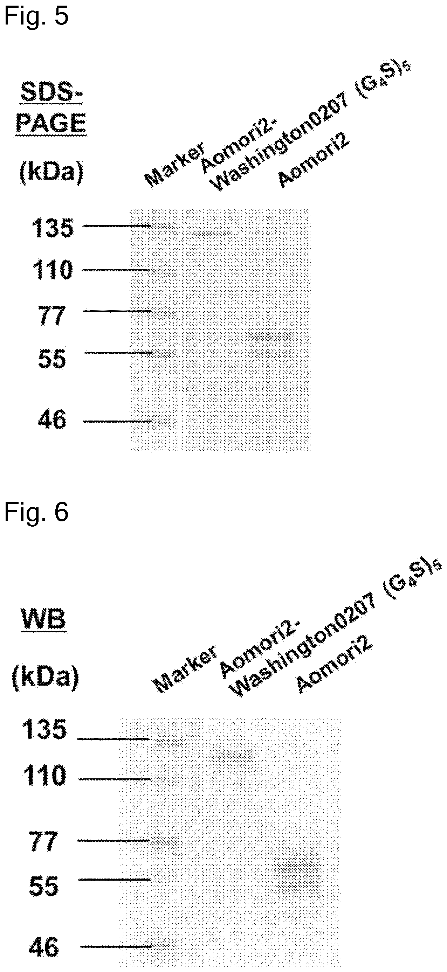

The results of SDS-PAGE analysis are shown in Fig. 5. The linker-connected VLPs that were newly prepared had a single band detected around 120 kDa (the lane of "Aomori2-Washington0207 (G₄S)₅" in Fig. 5). On the other hand, in the comparative control sample (the lane of "Aomori2" in Fig. 5), no band was detected around 120 kDa, and a band that seems to correspond to VP1 monomer proteins was detected around 60 kDa. Further, according to the results of Western blotting (Fig. 6), the band of the linker-connected VLPs around 120 kDa was detected by an antibody specific to GII.4 VP1. Accordingly, the band was thought to correspond to the protein of interest in which 2 VP1s (VP1s derived from Aomori2 and Washington0207) were connected by the G₄S linker. These results confirmed that the preparation of the linker-connected VLPs was reproducible.

The linker-connected VLP prepared in this example had higher purity than that prepared in Example 2. One reason for this was thought to be that the treatment by the sucrose cushion centrifugation was added during purification operation.

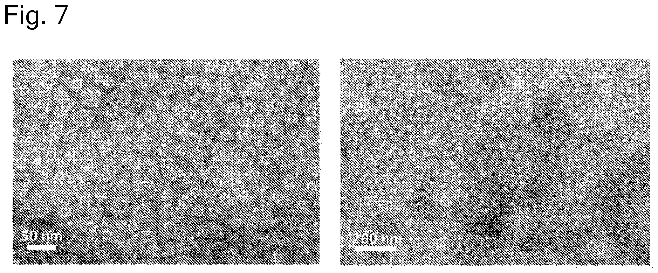

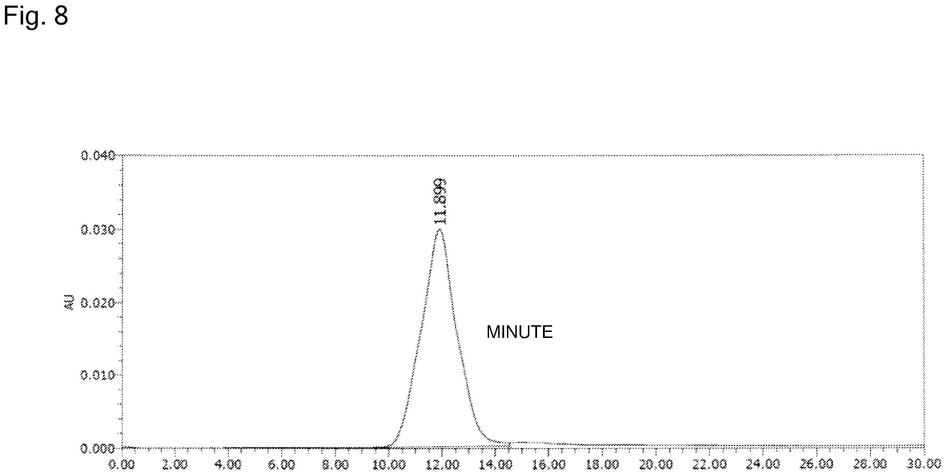

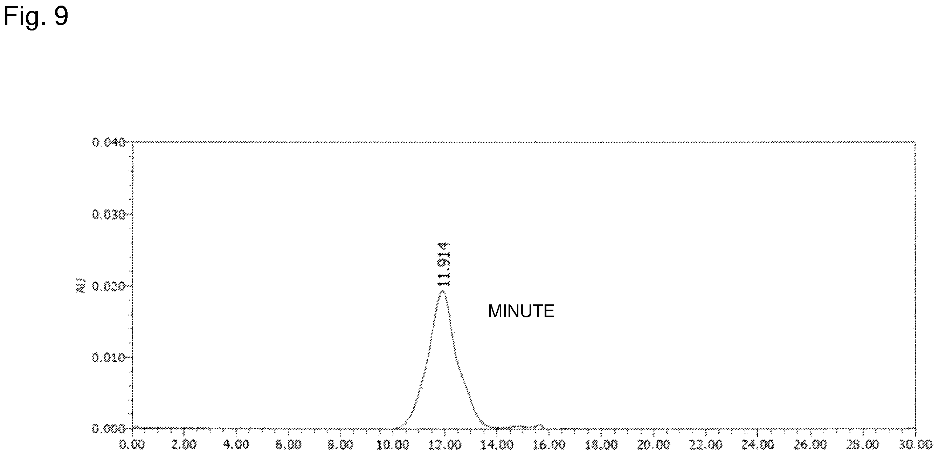

The structure of the linker-connected VLPs prepared in this example was analyzed using TEM. The images obtained from electron microscopy observation (Fig. 7) confirmed that the linker-connected VLPs formed particles in the same manner as in Example 4. When the SEC analysis was performed, the retention time of the linker-connected VLP (Fig. 8) and that of the comparable control sample (Fig. 9: Aomori2 VLP) were almost the same. In addition, the radius of gyration (rw) measured by MALS were 32.9 nm for the linker-connected VLP and 23.6 nm for the comparable control sample (Aomori2 VLP). These results confirmed that the linker-connected VLP has approximately the same particle size as the VLP formed from a single VP1.

### [Example 7] Confirmation of Antigenicity of Linker-Connected VLP

The antigenicity of the linker-connected VLP was evaluated by a receptor binding inhibition test using porcine gastric mucin (PGM). Refereeing to Haynes, J et al., Viruses 2019;11(5):392, a test was conducted as follows.

100 µl of PGM solution (1.0 µg/mL, PBS) prepared using commercially available PGM (catalog No. M1778, Sigma Aldrich Co. LLC) was added into each well of a well plate, and the well pate was allowed to stand at 25°C for 2 hours. Thereafter, the solution was removed, and 300 µl of washing buffer (PBS containing 0.05% Tween 20) was added thereto to wash each well (this washing operation was repeated 3 times). 200 µl of 5% skimmed milk-PBS solution was added to each well, and the mixture was allowed to stand overnight at 4°C.

200 µl each of 3 solutions (0.025 µg/ml each) each containing one of 3 VLPs (Aomori2 VLP, Washington0207 VLP, and Aomori2-Washington0207 linker-connected VLP (all prepared according to the method in Example 2)) were mixed with 200 µl of a mouse immune serum solution serially double-diluted from 40 times to 20480 times, and the mixture was allowed to stand for an hour at 25°C. The mixed solution was used as the VLP-mouse immune serum mixed solution. Note that the mouse immune serum was prepared from blood collected after each VLP (Aomori2 VLP, and Washington0207 VLP) was administered twice to 4 mice at 100 µg/head (4 lots for each VLP).

After each well of the well plate allowed to stand overnight at 4°C was washed (3 times) with washing buffer, 100 µl of the VLP-mouse immune serum mixed solution was added to each well, and the mixture was allowed to stand for an hour at 25°C. And then, the well plate was washed (3 times) with washing buffer, 100 µl of a primary antibody solution (in-house preparation) (dissolved in PBS containing 0.05% Tween 20) was added to each well and allowed to stand for an hour at 25°C. Note that the primary antibody solution was prepared from blood collected after each VLP (Aomori2 VLP, and Washington0207 VLP) was administered twice to rabbits at 1 mg/head. The plate was washed (3 times) with washing buffer, 100 µl of a secondary antibody solution (anti-rabbit IgG antibody, HRP conjugated (catalog# 65-6120, Invitrogen)) (dissolved in PBS containing 0.05% Tween 20) was added to each well and allowed to stand for an hour at 25°C. The plate was washed (3 times) with washing buffer, 100 µl of TMB solution (3,3',5,5'-tetramethyl benzidine) was added to each well and allowed to stand in the shade for 30 minutes at 25°C. 100 µl of stop solution was added thereto to stop the reaction. After the reaction stopped, the absorbance (OD) of the solution at a wavelength of 450 nm was measured.

The absorbance of the well to which the mixed solution of VLPs and PBS containing 0.05% Tween 20 and 5% skimmed milk (without mouse immune serum) was added was denoted as OD [no serum], the absorbance of the well without adding VLP-mouse immune serum mixed solution was denoted as blank, and the absorbance of the well to which the VLP-mouse immune serum mixed solution was added was denoted as OD [with serum] to calculate the binding-inhibitory activity using the following formula. Binding-inhibitory activity (%) = 100 - [(OD [with serum] - blank)/(OD [no serum] - blank)] × 100

The presence or absence of the receptor binding-inhibitory activity is determined based on the blocking titer (BT) 50 value. BT50 is a value of the maximum serum dilution factor at which the binding-inhibitory activity exceeds 50%. Statistical analysis based on the Mann-Whitney rank sum test was conducted on the BT50 values obtained for respective 3 VLPs (Aomori2 VLP, Washington0207 VLP, and
Aomori2-Washington0207 linker-connected VLP). The BT50 values were compared between the VLPs, and it was determined whether the linker-connected VLP has two types of antigenicity, based on the statistically significant difference.

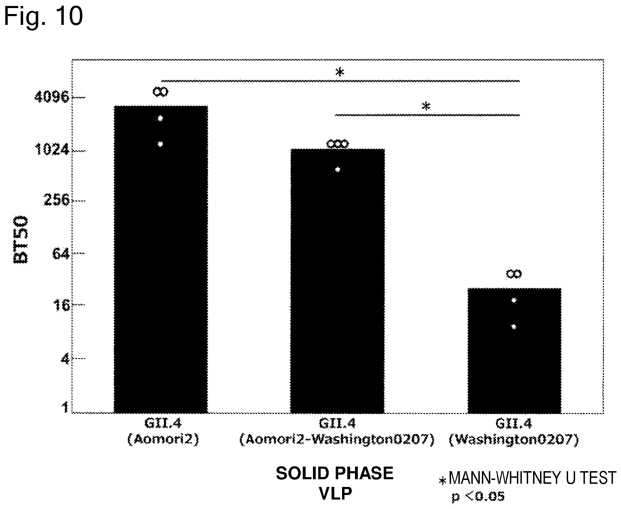

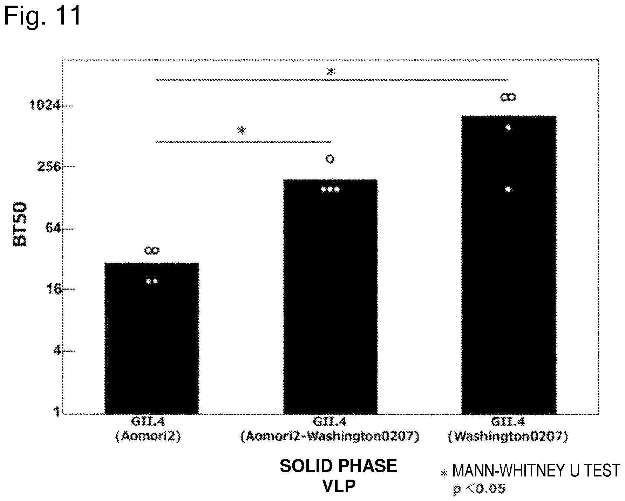

The results of the receptor binding inhibition test are shown in Figs. 10 and 11. Fig. 10 shows the results of the receptor binding-inhibitory activity against each VLP (Aomori2 VLP, Washington0207 VLP, and Aomori2-Washington0207 linker connected VLP), when using the mouse immune serum (4 lots) against Aomori2 VLP was used. Fig. 11 shows the results of the receptor binding-inhibitory activity against each VLP, when using the mouse immune serum (4 lots) against Washington0207 VLP was used.

As shown in Fig. 10, the mouse immune serum against Aomori2 VLP had a significantly lower value of the receptor binding-inhibitory activity against Washington0207 VLP. On the other hand, the mouse immune serum was confirmed to have the receptor binding-inhibitory activity against the linker connected VLP comprising Aomori2 VP1 at the same level as that against Aomori2 VLP.

In addition, as shown in Fig. 11, the mouse immune serum against Washington0207 VLP also showed a slight receptor binding-inhibitory activity against Aomori2 VLP, but it was significantly lower than those against Washington0207 VLP and the linker-connected VLP. The receptor binding-inhibitory activities against Washington0207 VLP and the linker-connected VLP were at a similar level.

Therefore, the linker-connected VLP according to the present invention is a chimeric VLP formed from 2 Norovirus VP1s connected to each other by a linker, and the chimeric VLP was thought to be a multivalent antigenic particle having the antigenicity of 2 VP1s.

## Claims

1. A virus-like particle, comprising an assembly of dimers of a first capsid protein and a second capsid protein,
wherein the first capsid protein and the second capsid protein are connected to each other by a linker.

2. The virus particle according to claim 1, wherein the linker is a GS linker.

3. The virus-like particle according to claim 2, wherein a C-terminus of the first capsid protein and an N-terminus of the second capsid protein are connected to each other by the GS linker.

4. The virus-like particle according to claim 2 or 3, wherein the GS linker is a (GGGGS)ₙ linker, where n is an integer of 1 or more.

5. The virus-like particle according to any one of claims 1 to 4, wherein the first capsid protein and the second capsid protein are derived from the same virus or different viruses.

6. The virus-like particle according to any one of claims 1 to 5, wherein the first capsid protein and the second capsid protein are derived from viruses of the *Caliciviridae* family.

7. A capsid protein dimer, wherein a first capsid protein and a second capsid protein are connected to each other by a linker.

8. The capsid protein dimer according to claim 7, wherein the linker is a GS linker.

9. The capsid protein dimer according to claim 8, wherein a C-terminus of the first capsid protein and an N-terminus of the second capsid protein are connected to each other by the GS linker.

10. The capsid protein dimer according to claim 8 or 9, wherein the GS linker is a (GGGGS)ₙ linker, where n is an integer of 1 or more.

11. The capsid protein dimer according to any one of claims 7 to 10, wherein the first capsid protein and the second capsid protein are derived from the same virus or different viruses.

12. The capsid protein dimer according to any one of claims 7 to 11, wherein the first capsid protein and the second capsid protein are derived from viruses of the *Caliciviridae* family.

13. A polynucleotide comprising a structure in which a polynucleotide that encodes a first capsid protein and a polynucleotide that encodes a second capsid protein are connected to each other through a polynucleotide that encodes a linker.

14. The polynucleotide according to claim 13, wherein the linker is a GS linker.

15. The polynucleotide according to claim 14, wherein the GS linker is a (GGGGS)ₙ linker, where n is an integer of 1 or more.

16. The polynucleotide according to any one of claims 13 to 15, wherein the first capsid protein and the second capsid protein are derived from the same virus or different viruses.

17. The polynucleotide according to any one of claims 13 to 16, wherein the first capsid protein and the second capsid protein are derived from viruses of the *Caliciviridae* family.

18. An expression vector comprising the polynucleotide according to any one of claims 13 to 17.

19. A host cell, into which the expression vector according to claim 18 is introduced.

20. A pharmaceutical composition comprising the virus-like particle according to any one of claims 1 to 6, or the capsid protein dimer according to any one of claims 7 to 12.

21. The pharmaceutical composition according to claim 20, wherein the pharmaceutical composition is used for induction of protective immunity.

22. The pharmaceutical composition according to claim 20 or 21, wherein the pharmaceutical composition is a vaccine.
